# NEUE EUROPÄISCHE PATENTSCHRIFT

(11) **EP 2 349 138 B3**
(45) Veröffentlichungstag dieser Patentschrift: **18.11.2015**
(45) Hinweis auf die Patenterteilung: 20.02.2013
(21) Anmeldenummer: 09736799.9
(22) Anmeldetag: 21.08.2009
(51) Int. Cl.: A61F 5/56

(54) **VERSTELLBARE UNTERKIEFERPROTRUSIONSSCHIENE ZUR BEHANDLUNG VON SCHNARCHEN UND OBSTRUKTIVER SCHLAFAPNOE**
ADJUSTABLE MANDIBULAR PROTRUSION SPLINT FOR TREATING SNORING AND OBSTRUCTIVE SLEEP APNEA
GOUTTIÈRE DE PROPULSION MANDIBULAIRE RÉGLABLE POUR TRAITER LE RONFLEMENT ET L'APNÉE OBSTRUCTIVE DU SOMMEIL

(30) Priorität: 06.09.2008 DE 202008011841 U
(43) Veröffentlichungstag der Anmeldung: 03.08.2011
(73) Patentinhaber: Toussaint, Winfried, 64625 Bensheim (DE)
(72) Erfinder: Toussaint, Winfried, 64625 Bensheim (DE)
(74) Vertreter: Kompter, Hans-Michael
(86) Internationale Anmeldenummer: PCT/DE2009/001179
(87) Internationale Veröffentlichungsnummer: WO 2010/025700

(56) Entgegenhaltungen:
- DE-C1- 10 216 242
- DE-U1-202005 015 106
- FR-A1- 2 887 140
- US-A- 6 109 265
- US-A1- 2007 283 967
- US-B1- 6 170 485
- US-B1- 6 418 933

## Beschreibung

### HINTERGRUND DER ERFINDUNG

### 1. TECHNISCHES GEBIET

Die Erfindung betrifft ein gebrauchsfertiges Set zur Herstellung einer verstellbaren, zweiteiligen Unterkieferprotrusionsschiene zur Behandlung von Schnarchen und/oder obstruktiver Schlafapnoe.

### 2. STAND DER TECHNIK

Schnarchen kann ein Symptom für das obstruktive Schlafapnoesyndrom sein, gekennzeichnet durch wiederholte und zahlreich austretende nächtliche Atemstillstände, die schwerwiegende gesundheitliche Komplikationen, wie z.B. Bluthochdruck, Herz-Kreislauferkrankungen, Gehirnschlag u.a. nach sich ziehen können. Durch das US Patent US 5,462,066 sowie die europäische Patentanmeldung EP 1 203 570 sind derartige zahnspangenartige Aufbissschienen zur Verhinderung des Schnarchens bekannt geworden, welche dazu dienen, den Unterkiefer geringfügig nach vorne zu verschieben, weil in dieser Stellung des Unterkiefers die Atemwege weiter geöffnet werden, so dass der Patient freier atmen kann, ohne zu schnarchen.

Die bekannten Aufbissschienen in Form eines einteiligen, zahnspangenartigen Mundstücks bestehen aus thermoplastischen Materialien mit zwei Bissrillen, welche bei Erwärmung formbar werden. Der Patient nimmt das erwärmte, noch nicht angepasste Mundstück in den Mund, um dann in den formbaren Kunststoff die Zähne des Untersowie des Oberkiefers in die entsprechende untere sowie obere Bissrille hineinzudrücken und durch Aufbeissen auf die Bissplatten der Bissrillen anzupassen. Dabei kühlt der Kunststoff ab und gewinnt seine feste Elastizität zurück, wonach nunmehr das Mundstück an den Patienten angepasst ist. Beim Anpassungsvorgang muss darauf geachtet werden, den Unterkiefer etwas nach vorne zu verschieben, um dauerhaft einen Vorschub (Protrusion) einzustellen. Die bekannten Aufbissschienen besitzen den Nachteil, dass eine einmal eingestellte Protrusion nur schwierig sich ändernden Bedürfnissen des Patienten angepasst werden kann, so dass sich mit der Zeit der anfänglich erzielte Effekt verschlechtert.

Weiterhin schlägt das US Patent US 5,868,138 eine dentale Vorrichtung zum Behandeln von Schnarchen und obstruktivem Schlafatemstillstand vor, welches ein Oberkieferteil, ein Unterkieferteil und ein Verbindungsmittel aufweist, wobei das Verbindungsmittel das untere Teil relativ zum oberen Teil in einer vorderen vorstehenden Position einstellbar hält.

Das deutsche Gebrauchsmuster DE 29 506 512 schlägt eine oral zu tragende Antischnarchvorrichtung aus einer Ober- und Unterkieferschiene vor, wobei beide Schienen mit einem flexiblen, in Längsrichtung nicht dehnbaren Zug versehen sind, der beim Absinken des Unterkiefers diesen in eine anteriore Lage bringt.

Dazu müssen individuell im Dentallabor nach Abdrucknahme Ober- und Unterkieferschienen angefertigt werden.

Das US Patent US 6,109,265 schlägt eine ebenfalls im Denallabor individuell zu fertigende zweiteilige Unterkieferprotrussionsschien vor, bei der die Ober- und Unterkieferschiene über ein ein elastisches Band ("elastic band"), vorzugsweise aus Polyurethan einer Härte von 55 bis 85A miteinander verbunden werden, wobei der Unterkiefer durch den von diesem Band ausgeübten elastischen Zug nach vorne verlagert wird.

Die deutsche Gebrauchsmusterschrift DE 20 2005015106 schlägteine zweiteilige Unterkieferprotrussionsschiene, welche jeweils ein thermoplastisches Füllmaterial enthält, bei der sich der Unterkiefervorschub durch eine sich im vorderen Bereich befindende Stellschraube stufenlos verstellen läßt.

Die US Patentanmeldung US 2007/0283977 beschreibt ebenfalls eine zweiteilige Unterkieferprotrussionsschiene, welche jeweils ein thermoplastisches Füllmaterial enthält, wobei das jeweils seitlich angebrachte Verbindungselement ein elastisches Element ("elastic element") ist.

Das US Patent US 6,418,933 B1 schlägt eine Unterkieferprotrusionsschiene mit Formgebenden und biegesteifen Schalen auf, deren inneren Oberflächen (26) individuell zur Aufnahme der Zähne des Benutzers ausgebildet sind. Diese inneren Oberflächen können mit einem weichen Gewebe ausgekleidet sein.

Die Herstellung dieser Unterkieferprotrusionsschiene umfasst unter anderem die Abnahme eines Gebissabdruckes, die Herstellung eines Gebissmodelles, die exakte Anpassung des Protrusionshalters sowie der Herstellung der Schiene auf Grundlage des Gebissmodelles.

Der vorliegenden Erfindung lag die Aufgabe zu Grunde, Schnarchern und Schlafapnoikern im Vergleich dazu eine in der Wirksamkeit vergleichbare Unterkieferprotrusionsschiene, die eine lange Lebensdauer aufweist und leicht zu handhaben ist, zur Verfügung zu stellen. Zudem sollte die zu entwickelnde Schiene problemlos vom Patienten selbst angepasst werden können, was eine erheblich Vereinfachung in der Handhabung erforderlich macht. Des weiteren sollte der Unterkiefervorschub der Unterkieferprotrusionsschiene leicht an die Bedürfnisse des Patienten angepasst werden können.

Diese Aufgabe wurde erfindungsgemäß gelöst durch die Bereitstellung eines gebrauchsfertigen Sets zur Herstellung einer zweiteiligen Unterkieferprotrusionsschiene umfassend ein Unter- (2) und ein Oberteil (3), aus einer jeweils in Gebrauch zum Unter- bzw. Oberkiefer geöffneten, Formgebenden und biegesteifen Schale (2a, 3a), welche jeweils ein thermoplastisches Füllmaterial (4, 5) enthält, das jeweils zahnspangenartig dem menschlichen Ober- und Unterkiefer nachformbar ist, wobei die beiden Schalen an ihren jeweiligen Außenseiten einen oder mehrere Fixierungsknöpfe zur Befestigung eines Protrusionshalters aufweisen, welcher jeweils an einem Fixierungsknopf der Unter- und der Oberkieferschale drehbar befestigt ist und den Unterkiefer in eine anteriore Lage bringt. Durch den Einsatz mehrerer Protrusionshalter unterschiedlicher Länge lässt sich der Unterkiefervorschub leicht den Bedürfnissen des jeweiligen Verwenders anpassen.

### KURZE ZUSAMMENFASSUNG DER ERFINDUNG

Gegenstand der Erfindung ist somit ein gebrauchsfertiges Set zur Herstellung einer zweiteiligen Unterkieferprotrusionsschiene (1) zur Verhinderung von Schnarchen und/oder von obstruktiver Schlafapnoe,
umfassend
(A) ein Unter- (2) und ein Oberteil (3), aus einer jeweils in Gebrauch zum Unter- bzw. Oberkiefer geöffneten, Formgebenden und biegesteifen Schale (2a, 3a), welche jeweils ein thermoplastisches Füllmaterial (4, 5) enthalten, das jeweils zahnspangenartig dem menschlichen Ober- und Unterkiefer nachformbar ist, wobei die beiden Schalen an ihren jeweiligen Außenseiten einen oder mehrere Fixierungsknöpfe zur Befestigung eines Protrusionshalters aufweisen, welcher jeweils an einem Fixierungsknopf der Unter- und der Oberkieferschale drehbar befestigt ist und den Unterkiefer in eine anteriore Lage bringt,
(B) zwei Sätze von jeweils zwei oder mehreren Protrusionshaltern (10) unterschiedlicher Länge,
wobei die Protrusionshalter gegenüber Dehnungen oder Stauchungen stabil sind.

Die Unterkieferprotrusionsschiene vermeidet die zeit- und kostenaufwendige individuelle Fertigung im Dentallabor nach vorheriger Zahnabdrucknahme durch einen Zahnarzt oder Kieferorthopäden. Zudem kann diese universelle Schiene problemlos von jedem Arzt - nicht nur von Zahnärzten- oder sogar vom Patienten selbst angepasst werden können, was eine erhebliche Vereinfachung in der Handhabung erforderlich macht.

Die Unterkieferprotrusionsschiene kann durch ein Verfahren umfassend die folgenden Schritte hergestellt werden:
(a) Formen der formgebenden und biegesteifen Schalen (2a, 3a) mit jeweils an den Außenseiten vorgeformten Fixierungsknöpfen im Spritzgussverfahren;
(b) Füllen der Schalen mit einem thermoplastisches Füllmaterial (4, 5) vorzugsweise per Spritzgussverfahren insbesondere bei Massenfertigung;
(c) Formen der Protrusionshalter
(d) Verbinden der Protrusionshalter mit den vorgeformten Fixierungsknöpfen.

Die universelle Unterkieferprotrusionsschiene besitzt den Vorteil, dass sie vergleichbar einer dentalen Schiene sehr fest auf beiden Kiefern sitzt bzw. haftet, da aufgrund der Materialbeschaffenheit der thermoplastischen Schienenfüllung problemlos eine sehr tiefe und gleichmäßige Impression sämtlicher Zähne erreicht wird. Begünstigt wird der feste Sitz auch durch die feste Einfassung des Thermoplasten durch die festen Außenwände eines biegesteifen Materials bestehend z.B. aus Polycarbonat. Des weiteren besitzt die Unterkieferprotrusionsschiene den Vorteil, dass sie unkompliziert ohne besondere Hilfsmittel von jedem Arzt oder sogar vom Patienten selbst innerhalb weniger Minuten angepasst werden kann. Aus diesem Grund genügt auch eine universelle Standardschiene passend für fast sämtliche Kieferformationen. Des weiteren ist in höchst vorteilhafter Weise eine individuelle Einstellung des Unterkiefervorschubs durch den Einsatz von Protrusionhalter unterschiedlicher Länge möglich. Durch die spezielle Konstruktion der Schiene wird erreicht, dass diese sehr zierlich ist und nach Anpassung der Abstand der oberen und unteren Frontzähne mit weniger als 3 mm nur sehr klein ist, was sich sehr positiv auf Tragekomfort und Akzeptanz auswirkt und auch darauf, dass die Schiene gleichzeitig für unterschiedlich große Kieferformationen geeignet ist.

### KURZE BESCHREIBUNG DER ABBILDUNGEN

Figur 1 zeigt eine perspektivische Ansicht von schräg oben und vorne einer Ausführungsform der universellen Protrusionsschiene (1);
Figur 2 zeigt eine schematische Darstellung einer ungefüllten Oberkieferschale (2a);
Figur 3 zeigt eine schematische Darstellung einer gefüllten Oberkieferschale (2a);
Figur 4 zeigt eine Aufsicht des Oberteils von oben;
Figur 5 zeigt eine schematische Seitenansicht des Oberteil;
Figur 6 zeigt eine schematische Ansicht eines Protrusionshalters;
Figur 6a zeigt einen Schnitt entlang der in Fig. 6 eingezeichneten Linie von "A" nach "A";
Figur 6b zeigt einen Schnitt entlang der in Fig. 6 eingezeichneten Linie von "B" nach "B";
Figur 7 zeigt eine schematische Ansicht eines Satzes von 8 Protrusionshaltern unterschiedlicher Länge, die in einem trapezförmigen Rahmen angebracht sind.
Figur 8 zeigt eine perspektivische Ansicht von schräg oben und vorne einer alternativen Ausführungsform der universellen Protrusionsschiene (1);
Figur 9 zeigt eine Aufsicht des Oberteils von oben der alternativen Ausführungsform der universellen Protrusionsschiene (1);
Figur 10 zeigt eine schematische Ansicht eines Satzes von 6 Protrusionshaltern unterschiedlicher Länge, die in einem trapezförmigen Rahmen angebracht sind für die alternativen Ausführungsform der universellen Protrusionsschiene.

### DETAILLIERTE BESCHREIBUG DER ERFINDUNG

Die Protrusionsschiene lässt sich aus bekannten Materialien universell massengefertigt im Spritzgussverfahren herstellen.

Der Begriff "Protrusionsschiene" bzw. " Unterkieferprotrusionsschiene" wie er vor- und nachstehend verwendet wird bezeichnet eine dentale Vorrichtung, welche es erlaubt, den Unterkiefer gegenüber dem Oberkiefer in eine leicht nach vorne gelagerte Position zu verschieben und dadurch den Querschnitt der oberen Atemwege zu vergrößern. Dies führt zu einer Reduktion des Schnarchens und von Atemaussetzern infolge eine obstruktiven Schlafapnoe.

Der Begriff "thermoplastisches Füllmaterial" wie er vor- und nachstehend verwendet wird bezeichnet ein Material, das sich in der Wärme, vorzugsweise unterhalb von 70 °C, vorzugsweise zwischen 40 und 65 °C plastisch verformen lässt und sich eng an eine vorgegebene Form anschmiegt und danach beim Abkühlen diese besagte Form beibehält. Geeignete Materialien sind u.a. z.B. Polymere und Kopolymere oder Gemische davon aus der Gruppe der Polyethylene (PE), Polyvinylacetate (PVA), Acrylate und Methacrylate, bevorzugt Kopolymere aus PE und PVA, wie sie zum Beispiel unter der Marke Elvax® der Firma DuPont erhältlich sind. Bevorzugt sind solche PE-PVA Kopolymere, deren PVA Gehalt von 20 bis 40, insbesondere 25 bis 35 % beträgt.

Der Begriff "Formgebende und biegesteife Schale" wie er vor- und nachstehend verwendet wird bezeichnet einen "U- bzw. hufeisenförmigen" Formkörper, der eine an den Enden offene oder geschlossene Schale oder Wanne ausbildet. In der Regel bestehtdieser Formkörper aus einem unter physiologischen Bedingungen inerten und stabilen Material wie zum Beispiel duroplastischen Kunststoffen, wie zum Beispiel Polytetrafluoroethylen (PTFE, Teflon®), oder Polykarbonaten oder aus Elastomeren wie zum Beispiel Polyacrylaten.

Der Begriff "Fixierungsknopf" wie er vor- und nachstehend verwendet wird bezeichnet eine stabförmige Erhebung mit einer endständigen Verdickung, die jeweils an der Seite der Unter- bzw. Oberkieferschale angebracht sind, und an denen der Protrusionshalter reversibel befestigt werden kann. In der Regel ist der Fixierungsknopf so ausgestaltet, dass die endständige Verdickung in eine Aussparung des Protrusionshalters einrastet und ihn um die Längsachse des Fixierungsknopfes drehbar fixiert.

Der Begriff "Protrusionshalter" wie er vor- und nachstehend verwendet wird bezeichnet ein Verbindungselement zwischen der Ober- und der Unterkieferschale, welches es ermöglicht, dass der Unterkiefer im getragenen Zustand in eine anteriore Lage gebracht wird. Er weist endständige Aussparungen auf, in die jeweils ein Fixierungsknopf einrasten kann. Die Geometrie des Protrusionshalters an sich ist unkritisch; er sollte lang genug sein, um den Unterkiefer in eine anteriore Lage zu bringen. Er sollte gegenüber Dehnungen oder Stauchungen stabil sein. Er muss relativ dünn sein, um einen guten Tragekomfort zu gewährleisten.

Die vor- und nachstehend verwendeten Angaben hinsichtlich der Geometrie oder räumlichen Anordnung der Protrusionsschiene oder von Teilen davon orientieren sich an den Gegebenheiten der Protrusionsschiene bei ihrer sinngemäßen Verwendung, d.h. nach Einsetzen derselben auf die obere und untere Zahnreihe der betroffenen Person: "in Längsrichtung" bedeutet in Richtung der Mundöffnung ("anterior") oder des Rachens ("posterior"); "nach vorne" (anterior) bedeutet in Richtung der Mundöffnung; "mittig" bedeutet im Bereich der Schneidezähne; "hinten" bedeutet im Bereich der hinteren Backenzähne; "seitlich" bedeutet im Bereich zwischen den Prämolaren und den hintern Molaren.

Vorteilhafte Ausgestaltungen der Erfindung sind solche Sets zur Herstellung von Unterkieferprotrusionsschienen, wobei
(a) die Fixierungsknöpfe einen Durchmesser von 2,0 bis 5,0 mm, vorzugsweise von 3,0 bis 4,5 mm insbesondere etwa 4 mm aufweisen;
(b) der Abstand zwischen der Innenseite des Bodens der Oberkieferschale (11) und der Innenseite des Bodens der Unterkieferschale (12), im zusammengesetzten Zustand weniger als 8 mm, vorzugsweise 1 bis 5 mm, insbesondere etwa 2 mm beträgt;
(c) der Abstand der Zähne bei angelegter Schiene im Frontzahnbereich bei etwa 2 bis 4 mm liegt. Besonders bevorzugt ist ein Abstand der Zähne im Frontzahnbereich von etwa 3 mm bei angelegter Schiene. Dies führt im Regelfall zu einem leichterem und dabei vollständigerem Schließen des Mundes auch bei kleinen Kiefergrößen ohne Muskelanspannung. Dadurch resultieren höherer Tragekomfort und damit auch eine bessere Akzeptanz (Compliance).
(d) das Füllmaterial (4, 5) aus einem oder mehreren Kopolymeren aus Polyethylen und Polyvinylacetat besteht;
(e) die Formgebende und biegesteife Schale (2a, 3a) aus Polycarbonat besteht;
(f) die Protrusionshalter aus einem elastischen und reißfesten Material, insbesondere aus Polyoxmethylen (POM, z.B. Ultraform / BASF) bestehen;

Vorzugsweise sind die Protrusionshalter, deren Länge kürzer ist als der Abstand zwischen zwei Fixierungsknöpfen, jeweils mit dem vorderen Fixierungsknopf des Oberteils und dem hinteren Fixierungsknopf des Unterteils verbunden sind, wobei der Unterkiefer durch Zug in eine anteriore Lage gebracht wird; vorzugsweise wobei die biegesteifen Schalen (2a, 3a) jeweils an ihren beiden Außenseiten jeweils zwei Fixierungsknöpfe in einem Abstand von 20,0 bis 30,0 mm, vorzugsweise von 22,0 bis 28,0 mm, insbesondere von etwa 26,7 mm aufweisen und/oder die Protrusionshalter eine Gesamtlänge von 15,0 bis 35,0 mm, vorzugsweise 17,0 bis 31,0 mm aufweist.

In einer alternativen Ausführungsform sind die Protrusionshalter, deren Länge größer ist als der Abstand zwischen zwei Fixierungsknöpfen, jeweils mit dem hinteren Fixierungsknopf des Oberteils und dem vorderen Fixierungsknopf des Unterteils verbunden sind, wobei der Unterkiefers durch Druck in eine anteriore Lage gebracht wird; vorzugsweise wobei die biegesteifen Schalen (2a, 3a) jeweils an ihren beiden Außenseiten jeweils zwei Fixierungsknöpfe in einem Abstand von 10,0 bis 20,0 mm , vorzugsweise von 12,5 bis 18,0 mm, insbesondere von etwa 15,6 mm aufweisen und/oder die Protrusionshalter jeweils eine Gesamtlänge von 15,0 bis 30,0 mm, vorzugsweise 17,0 bis 27,0 mm aufweisen.

Die Herstellung der universellen Unterkieferprotrusionsschiene an sich ist einfach:
(a) man formt die Formgebenden und biegesteifen Schalen (2a, 3a) mit jeweils auf den Außenseiten vorgeformten Fixierungsknöpfen im Spritzgußverfahren;
(b) füllt die Schalen mit einem thermoplastischen Füllmaterial (4, 5) ebenfalls per Spritzgussverfahren bei Massenfertigung bzw. in besonderen Einzelfällen auch manuell hergestellt; und
(c) formt die erforderlichen Protrusionshalter jeweils paarweise, und
(d) verbindet die Protrusionshalter mit den vorgeformten Fixierungsknöpfen.

Das verwendete Polymer oder Co-Polymer des thermoplastischen Füllmaterials kann, abgesehen von der üblichen Massenfertigung im Spritzgussverfahren, auch in besonderen Einzelfällen nach Erhitzen in Granulatform im Wasserbad manuell zu einer homogenen plastischen Masse geformt und dann in leere ungefüllte Schalen eingedrückt werden. Wenn man anschließend diese Schalen mit ihrer Füllung in heißem, vorzugsweise kochendem Wasser etwa 2 bis 4 Minuten erhitzt und nach kurzem Abkühlen die plastische Masse in den Schalen gleichmäßig verteilt und sodann aushärten lässt, kann man so eine voll funktionsfähige Unterkieferprotrusionsschiene herstellen. Die polymere Masse haftet absolut fest auf der Kontaktfläche aus Polycarbonat. Falls einem Arzt oder einem Patienten beim ersten Versuch die Anpassung durch Einbeißen an der falschen Stelle misslingen sollte, kann auf diese Art und Weise die Schiene sehr einfach wieder in den Neuzustand zurückversetzt werden.

Sollte weiterhin nach langer Tragezeit über viele Monate die Haftung der Schiene an Zähnen und Kiefern nachlassen, kann man so ebenfalls problemlos die ursprüngliche Schienenfüllung vollständig wiederherstellen und dann die Anpassung erneut vornehmen. Die Unterkieferprotrusionsschiene kann somit gegebenenfalls nach zwischenzeitlichen Reparaturen über einen sehr langen Zeitraum verwendet werden.

Ausführungsbeispiele der Erfindung sind in den Zeichnungen dargestellt und werden im folgenden näher beschrieben.

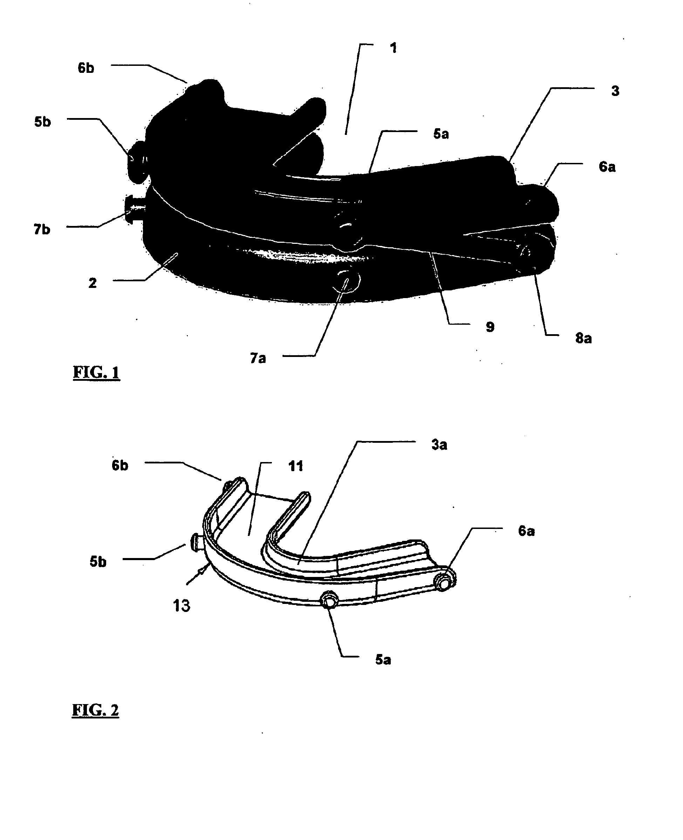

Figur 1 zeigt eine perspektivische Ansicht von schräg oben und vorne einer Ausführungsform der universellen Protrusionsschiene (1) gebildet aus einem Unterteil (2) und einem Oberteil (3), die jeweils aus einer Schale (2a, 3a) und einem nicht dargestellten Füllmaterial (4) gebildet werden. Weiterhin weisen sowohl das Unterteil (2) als auch das Oberteil (3) der Protrusionsschiene (1) jeweils auf beiden Seitenbereichen zwei Fixierungsköpfe (5a, 6a, 7a, 8a, 5a; 6b, 7b, 8b) wobei die beiden vorderen Fixierungsknöpfe des Oberteils (5a, 5b) jeweils über einen Protrusionshalter (9) mit den beiden hinteren Fixierungsknöpfen (8a, 8b) des Unterteils verbunden sind.

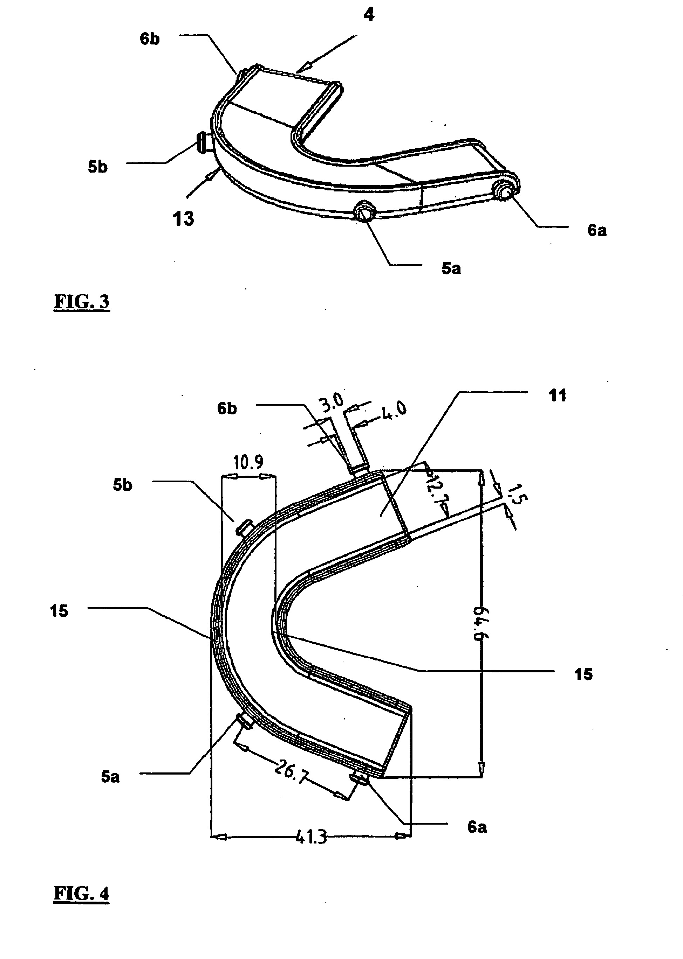

Figur 2 zeigt eine schematische Darstellung einer ungefüllten Oberkieferschale (3a); mit den vier Fixierungsköpfen (5a, 6a, 5b, 6b). Wie aus den Fig3 zu erkennen ist, überragt das thermoplastische Material (4) jeweils die Höhe der biegefesten Schalen, vorzugsweise um 0,5 bis 3 mm, insbesondere um 1 bis 1,5 mm. Dies führt zu einer erhöhten Haftung der Zähne und gleichzeitig zu einem Schutz des Zahnfleisches gegenüber Verletzung durch die Kanten der biegefesten Schalen.

Figur 4 zeigt eine Aufsicht des Oberteils (3a) von oben, wobei die Abmessungen der einzelnen Bereiche der Oberschale angegeben sind. So beträgt der Abstand der beiden Außenwangen (15) der Schale an der posterioren Seite 12,7 mm und im anterioren Berewich 10.9 mm. Die Wandstärke der Außenwange (15) beträgt etwa 1,5 mm. Der Abstand zwischen der vordersten Position der Schale und der hintersten Position beträgt etwa 41,3 mm und der Abstand zwischen den beiden äußeren posterioren Enden der Schale beträgt etwa 64,6 mm. Der Abstand zwischen dem vorderen Fixierungsknopf (5a, 5b) und dem jeweiligen hinteren Fixierungsknopf (6a, 6b)beträgt etwa 26.7 mm.

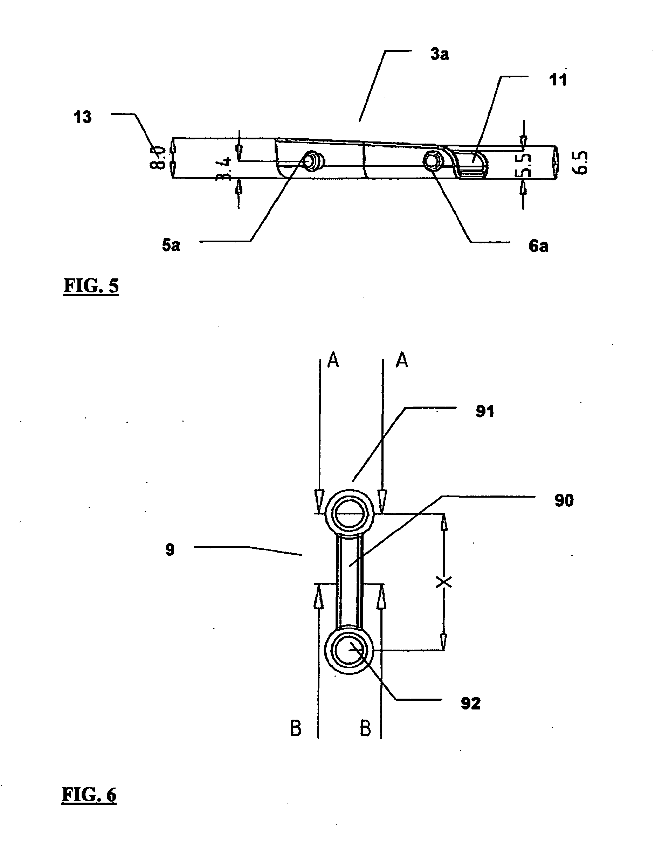

Figur 5 zeigt eine schematische Seitenansicht des Oberteils (3a), wobei die Höhe der Außenwange (15) angegeben sind. Diese beträgt im anterioren Bereich etwa 8, und im posterioren Bereich etwa 5,5 bis 6,5 mm.

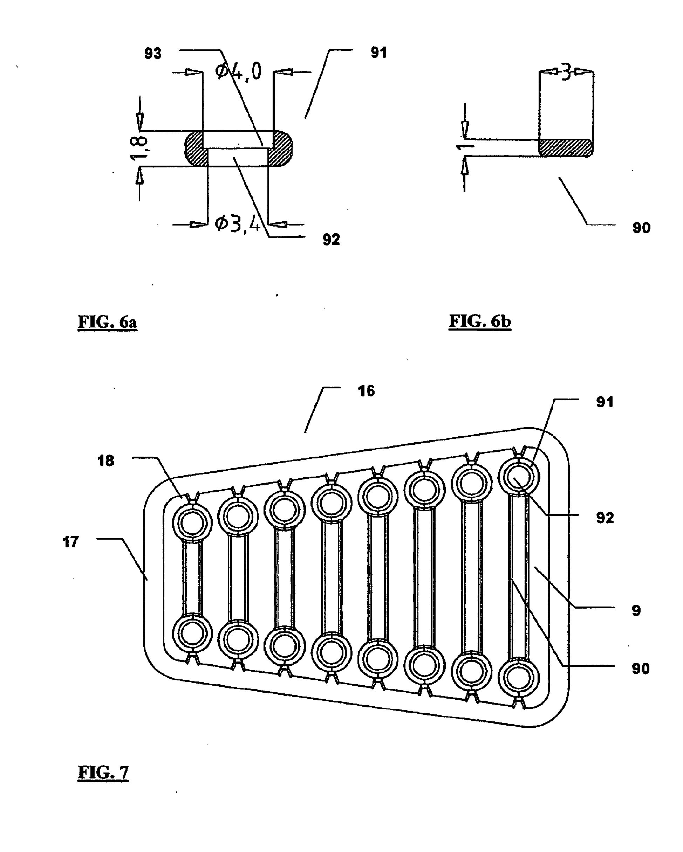

Figur 6 zeigt eine schematische Ansicht eines Protrusionshalters (9) mit den beiden endständigen Befestigungselementen (91) und deren Ringförmigen Aussparungen (92).

Figur 6a zeigt einen Schnitt entlang der in Fig. 6 eingezeichneten Linie durch ein Befestigungselement (91) von "A" nach "A" und gibt die jeweiligen Abmessungen desselben an. Deutlich ist die Abstufung (93) der ringförmigen Aussparung (92) zu erkennen, die zu einem besseren Einraten des Befestigungselementes (91) in einen der Fixierungsknöpfe ermöglicht. Das Befestigungselement ist etwa 1,8 mm stark und die ringförmige Aussparung weist an der der jeweiligen Schale zugewandten Seite einen Innendurchmesser von etwa 3,4 mm und and der abgewandten Seite einen Innendurchmesser von etwa 4,0 mm auf

Figur 6b zeigt einen Schnitt entlang der in Fig. 6 eingezeichneten Linie durch die Stange (91) und gibt die jeweiligen Abmessungen desselben an. von "B" nach "B"; die Stange (91) ist etwa 1 mm stark und etwa 3mm breit.

Figur 7 zeigt eine schematische Ansicht eines Satzes (16) von 8 Protrusionshaltern (9) unterschiedlicher Länge, die in einem trapezförmigen Rahmen (17) über Befestigungselemente (18) angebracht sind. Der jeweils benötigte Protrussionshalter lässt sich leucht durch drehen oder knicken aus dem Halter entfernen.

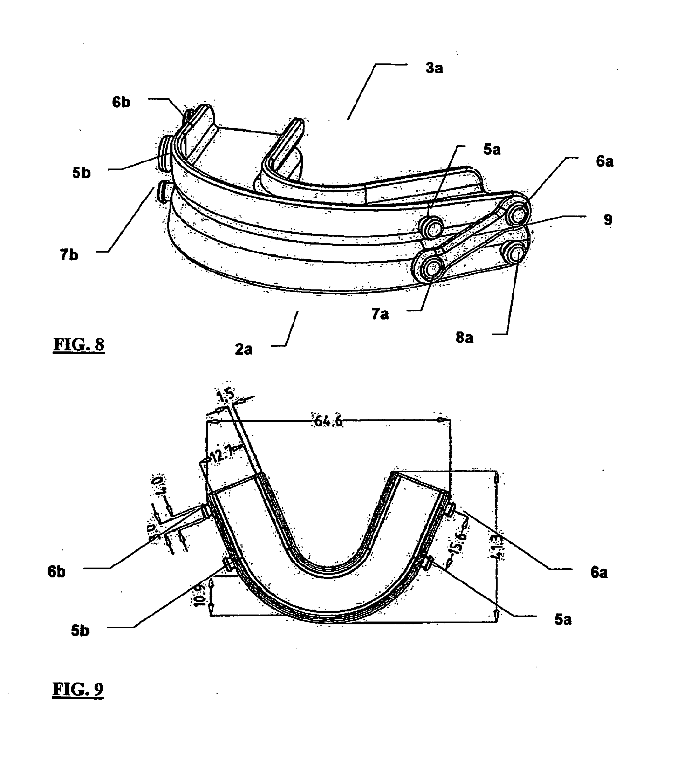

Figur 8 zeigt eine perspektivische Ansicht von schräg oben und vorne einer alternativen Ausführungsform der universellen Protrusionsschiene (1) gebildet aus einem Unterteil (2) und einem Oberteil (3), die jeweils aus einer Schale (2a, 3a) und einem nicht dargestellten Füllmaterial (4) gebildet werden. Weiterhin weisen sowohl das Unterteil (2) als auch das Oberteil (3) der Protrusionsschiene (1) jeweils auf beiden Seitenbereichen zwei Fixierungsköpfe (5a, 6a, 7a, 8a, 5a; 6b, 7b, 8b) wobei die beiden hinteren Fixierungsknöpfe des Oberteils (6a, 6b) jeweils über einen Protrusionshalter (9) mit den beiden vorderen Fixierungsknöpfen (7a, 7b) des Unterteils verbunden sind.

Figur 9 zeigt eine Aufsicht des Oberteils von oben der alternativen Ausführungsform der universellen Protrusionsschiene (1) von Fig. 8, wobei die Abmessungen der einzelnen Bereiche der Oberschale angegeben sind. Es fällt auf, dass bei dieser Ausführungsform die Abstände zwischen den vorderen Fixierungsknöpfen (5a, 5b) und den hinteren Fixierungsknöpfen (6a, 6b) kürzer sind, nämlich nur etwa 15,6 mm, alsbei der Ausführungsform der Fig. 3.

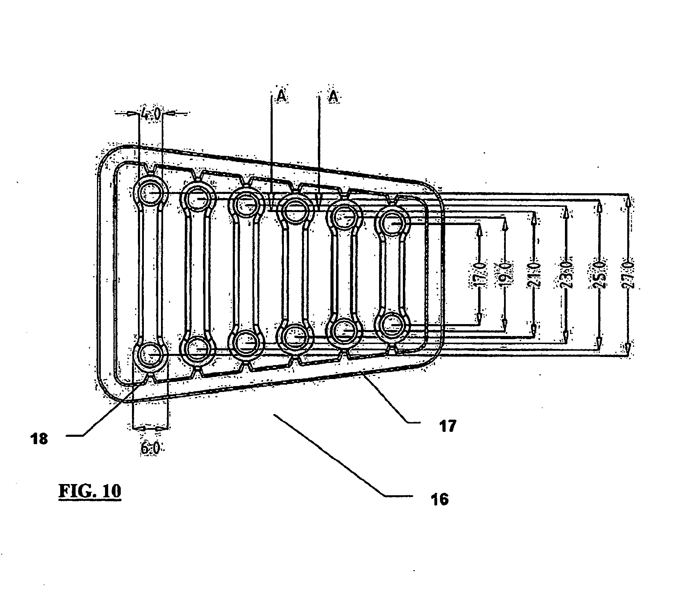

Figur 10 zeigt eine schematische Ansicht eines Satzes von 6 Protrusionshaltern unterschiedlicher Länge, die in einem trapezförmigen Rahmen angebracht sind für die alternativen Ausführungsform der universellen Protrusionsschiene.

Der Abstand zwischen den inneren Böden der beiden Schalen im Frontzahnbereich ("Zahnabstand") beträgt weniger als 8 mm, vorzugsweise 1 bis 5 mm, insbesondere etwa 2 mm. Im Bereich der Schneidezähne weist die Protrusionsschiene vor Anpassung an das Gebiss eine Gesamthöhe von weniger als 20 mm auf, vorzugsweise 10 bis 20 mm, insbesondere etwa 16 mm auf.

Die in den Figuren 2 bis 5 und 9 dargestellten Detailansichten des Oberteils (3a) verdeutlichen den Aufbau und die Funktion dieses Teils der Protrusionsschiene.

Der Abstand der beiden Außenwangen am hinteren Ende des Oberteils (maximale Breite der Unterkieferschiene) beträgt 60 bis 70 mm, vorzugsweise 62 bis 68 mm, insbesondere etwa 65 mm. Der Abstand zwischen der Außenwange (33) im Bereich der Schneidezähne und einer gedachten Linie zwischen den hinteren Enden der Innenwange (43) (maximale Länge der Unterkieferschiene) beträgt 38 bis 45 mm, insbesondere etwa 41,3 mm (vgl. Fig. 9). Zur Herstellung der biegestabilen Oberschale (3a) werden 3,0 bis 4,0 cm³ Polycarbonat verwendet.

Zur Herstellung der Füllung der Oberschale werden 5,0 bis 12,0 cm³ eines Copolymers aus PE und PVA verwendet.

Das Anpassen der universellen Protrusionsschiene an den Kiefer der betroffenen Person ist einfach. Die Anpassung kann im Regelfall von jedem Arzt gleich welcher Fachrichtung, dessen instruiertem Personal oder zumeist auch von der betroffenen Person alleine mit Hilfe eines Spiegels durchgeführt werden.
Bei Vorliegen von Zahnfehlstellungen, anamnestischen Kiefergelenksbeschwerden oder Zahnerkrankungen wie z.B. Parodontose u.a. ist es ratsam, vor Anpassung einen Zahnarzt zu konsultieren..

Bei der Anpassung der Schiene erwärmt man zuerst das Oberteil (3) auf eine Temperatur oberhalb der Erweichungstemperatur des Füllmaterials (4), vorzugsweise in einem Wasserbad bei einer Wassertemperatur von oberhalb 50 °C. Danach passt man das Oberteil an die Zahnreihe des Oberkiefers an, indem man die Zähne in das weiche, plastische und noch warme Füllmaterial gleichmäßig tief eindrückt. Anschließend lässt man das Füllmaterial kurz ca. 30 - 60 Sekunden im Mund abkühlen und dann endgültig in einem kalten Wasserbad aushärten.

Danach erwärmt man das Unterteil (2) entsprechend und verbindet es mit dem bereits angepassten Oberteil mit Hilfe zweier Protrussionshalter gleicher Länge, setzt das ausgeformte Oberteil in den Oberkiefer ein und drückt vorsichtig die Zähne des Unterkiefers in das weiche Füllmaterial (4) des Unterteils ein. Nach Aushärten ist die vorgefertigte Unterkieferprotrusionsschiene einsatzbereit.

Die Unterkieferprotrusionsschiene beseitigt oder reduziert Schnarchen und Atemaussetzer infolge einer obstruktiven Schlafapnoe. Durch Ihre zierliche Größe weist sie einen hohen Tragekomfort auf. Sie vermittelt einen festen, sicheren Halt für die umfassten Zähne, kann durch Austausch der Protrusionshalter in kleinen Stufen (inkrementell) hinsichtlich des Unterkiefervorschubs an die Bedürfnisse des Patienten angepasst werden, schützt die Zähne gegen nächtliches Zähneknirschen und das Zahnfleisch vor Verletzungen.

## Patentansprüche

1. Gebrauchsfertiges Set zur Herstellung einer zweiteiligen Unterkieferprotrusionsschiene (1) zur Verhinderung von Schnarchen und/oder von obstruktiver Schlafapnoe,
umfassend
(A) ein Unter- (2) und ein Oberteil (3), aus einer jeweils in Gebrauch zum Unter- bzw. Oberkiefer geöffneten, Formgebenden und biegesteifen Schale (2a, 3a), welche jeweils ein thermoplastisches Füllmaterial (4, 5) enthalten, das jeweils zahnspangenartig dem menschlichen Ober- und Unterkiefer nachformbar ist, wobei die beiden Schalen an ihren jeweiligen Außenseiten einen oder mehrere Fixierungsknöpfe zur Befestigung eines Protrusionshalters aufweisen, welcher jeweils an einem Fixierungsknopf der Unter- und der Oberkieferschale drehbar befestigt ist und den Unterkiefer in eine anteriore Lage bringt,
(B) zwei Sätze von jeweils zwei oder mehreren Protrusionshaltern (10) unterschiedlicher Länge,
**dadurch gekennzeichnet, dass** die Protrusionshalter gegenüber Dehnungen oder Stauchungen stabil sind.

2. Gebrauchsfertiges Set nach Anspruch 1, **dadurch gekennzeichnet, dass** die Fixierungsknöpfe einen Durchmesser von 2,0 bis 5,0 mm, vorzugsweise von 3,0 bis 4,5 mm insbesondere etwa 4 mm aufweisen.

3. Gebrauchsfertiges Set nach einem der Ansprüche 1 oder 2, **dadurch gekennzeichnet, dass** der Abstand zwischen der Innenseite des Bodens der Oberkieferschale (23) und der Innenseite des Bodens der Unterkieferschale (22), im zusammengesetzten Zustand weniger als 8 mm, vorzugsweise 1 bis 5 mm, insbesondere etwa 2 mm beträgt.

4. Gebrauchsfertiges Set nach einem der Ansprüche 1 bis 3, **dadurch gekennzeichnet, dass** der Abstand der Zähne bei angelegter Schiene im Frontzahnbereich 2 bis 4 mm, vorzugsweise etwa 3 mm beträgt.

5. Gebrauchsfertiges Set nach einem der Ansprüche 1 bis 4, **dadurch gekennzeichnet, dass** das Füllmaterial (4, 5) ein biokompatibles, toxikologisch unbedenkliches Polymer oder Kopolymer ist, das sich in der Wärme, vorzugsweise unterhalb von 70 °C, insbesondere zwischen 40 und 65 °C plastisch verformen lässt und sich eng an eine vorgegebene Form anschmiegt und danach beim Abkühlen diese besagte Form beibehält.

6. Gebrauchsfertiges Set nach einem der Ansprüche 1 bis 5, **dadurch gekennzeichnet, dass** die Protrusionshalter aus Polyoxymethylen bestehen.

7. Gebrauchsfertiges Set nach einem der Ansprüche 1 bis 6, **dadurch gekennzeichnet, dass** die Protrusionshalter, deren Länge kürzer ist als der Abstand zwischen zwei Fixierungsknöpfen, jeweils mit dem vorderen Fixierungsknopf des Oberteils und dem hinteren Fixierungsknopf des Unterteils zu verbinden sind, wobei der Unterkiefer durch Zug in eine anteriore Lage gebracht wird.

8. Gebrauchsfertiges Set nach Anspruch 7, **dadurch gekennzeichnet, dass** die biegesteifen Schalen (2a, 3 a) jeweils an ihren beiden Außenseiten jeweils zwei Fixierungsknöpfe in einem Abstand von 20,0 bis 30,0 mm , vorzugsweise von 22,0 bis 28,0 mm, insbesondere von etwa 26,7 mm aufweisen.

9. Gebrauchsfertiges Set nach einem der Ansprüche 1 bis 8, **dadurch gekennzeichnet, dass** die Protrusionshalter jeweils eine Gesamtlänge von 15,0 bis 35,0 mm, vorzugsweise 17,0 bis 31,0 mm aufweisen.

10. Gebrauchsfertiges Set nach einem der Ansprüche 1 bis 6, **dadurch gekennzeichnet, dass** die Protrusionshalter, deren Länge größer ist als der Abstand zwischen zwei Fixierungsknöpfen, jeweils mit dem hinteren Fixierungsknopf des Oberteils und dem vorderen Fixierungsknopf des Unterteils zu verbinden sind, wobei der Unterkiefers durch Druck in eine anteriore Lage gebracht wird.

11. Gebrauchsfertiges Set nach Anspruch 10, **dadurch gekennzeichnet, dass** die biegesteifen Schalen (2a, 3 a) jeweils an ihren beiden Außenseiten jeweils zwei Fixierungsknöpfe in einem Abstand von 10,0 bis 20,0 mm , vorzugsweise von 12,5 bis 18,0 mm, insbesondere von etwa 15,6 mm aufweisen.

12. Gebrauchsfertiges Set nach einem der Ansprüche 1 bis 6, **dadurch gekennzeichnet, dass** die Protrusionshalter jeweils eine Gesamtlänge von 15,0 bis 30,0 mm, vorzugsweise 17,0 bis 27,0 mm aufweisen.

13. Gebrauchsfertiges Set nach einem der Ansprüche 1 bis 12, **dadurch gekennzeichnet, dass** der Protrusionshalter jeweils an beiden Enden eine ringförmige Aussparung zur Aufnahme jeweils eines Fixierungsknopfes an der jeweiligen biegesteifen Schale (2a, 3a) aufweist.

14. Gebrauchsfertiges Set nach Anspruch 13, **dadurch gekennzeichnet, dass** die ringförmige Aussparung abgestuft ist und auf der einen Seite einen Innendurchmesser von 3,8 bis 4,2 mm und auf der anderen Seite einen Innendurchmesser von 3,1 bis 3,6 mm aufweist.

## Claims

1. A ready-to-use kit for producing a two-part mandibular protrusion splint (1) for preventing snoring and/or obstructive sleep apnea, comprising
(A) a lower part (2) and an upper part (3), each of which includes a shaping and flexurally rigid mandibular tray (2a, 3a) which both are open toward the mandibula or the maxilla, respectively, during use; and each of trays respectively comprises a thermoplastic filler material (4, 5) which can be shaped in a manner of a dental brace for respectively fitting to the maxilla and the mandibula; wherein each of the trays comprise one or more fixing knobs, on the respective outer surfaces thereof, for fastening a protrusion holder, which is rotatably fastened to a fixing knob of each of the mandibular tray and the maxillar tray and brings the mandibula into an anterior position,
(B) two sets of two or more protrusion holders (10) of different lengths, **characterized in that** the protrusion holders each are stable towards stretching or compression.

2. The ready-to-use kit according to claim 1, **characterized in that** the fixing knobs exhibits a diameter of from 2.0 to 5.0 mm, preferably of from 3.0 to 4.5 mm, in particular of about 4 mm.

3. The ready-to-use kit according to of the claims 1 or 2, **characterized in that** the distance between the inside of the bottom of the rigid maxillary tray (23) and the inside of the bottom of the rigid mandibular tray (22), in an assembled state, is less than 8 mm, preferably 1 to 5 mm, in particular about 2 mm.

4. The ready-to-use kit according to one of the claims 1 to 3, **characterized in that** the distance of the teeth with the mandibular protrusion splint in place, is from 2 to 4 mm, in particular about 3 mm, in the anterior tooth region.

5. The ready-to-use kit according to one of the claims 1 to 4, **characterized in that** the filling material (4, 5) is a biocompatible toxicologically acceptable polymer or copolymer which is plastically deformable below 70 °C, preferably between 40 and 65 °C, so that it closely adapts to a predefined form and then retains the specified form upon cooling.

6. The ready-to-use kit according to one of the claims 1 to 5, **characterized in that** the protrusion holders consist of polyoxymethylene.

7. The ready-to-use kit according to one of the claims 1 to 6, **characterized in that** the protrusion holders, whose lengths are shorter than the distance between two fixing knobs, are each connected with a front fixing knob of the upper part and a rear fixing knob of the lower part, whereby the lower jaw is placed into an anterior position by tension.

8. The ready-to-use kit according to claim 7, **characterized in that** each of the rigid trays (2a, 3a) have two-fixing knobs on their outer surfaces, which have a distance from each other of from 20.0 to 30.0 mm, preferably of from 22.0 to 28.0 mm, in particular of about 26.7 mm.

9. The ready-to-use kit according to one of claims 1 to 8, **characterized in that** each protrusion holder has a total length of from 15.0 to 35.0 mm, preferably of from 17.0 to 31.0 mm.

10. The ready-to-use kit according to one of the claims 1 to 6, **characterized in that** the protrusion holders, whose lengths are longer than the distance between two fixing knobs, are each connected with the rear fixing knob of the upper part and the front fixing knob of the lower part, whereby the lower jaw is placed into an anterior position by pressure.

11. The ready-to-use kit according to claim 10, **characterized in that** the rigid trays (2a, 3a) each have two fixing knobs on their outer surfaces, which have a distance from each other of from 10.0 to 20.0 mm, preferably of from 12.5 to 18.0 mm, in particular of about 15.6 mm.

12. The ready-to-use kit according to one of the claims 1 to 6, **characterized in that** each protrusion holder has a total length from 15.0 to 30.0 mm, preferably from 17.0 to 27.0 mm.

13. The ready-to-use kit according to one of the claims 1 to 12, **characterized in that** each protrusion holder has, at both ends thereof, an annular recess for receiving a respective fixing knob on the rigid trays (2a, 3a).

14. The ready-to-use kit according to claim 13, **characterized in that** the annular recess is step like and has, on one side, an inner diameter of from 3.8 to 4.2 mm and, on the other side, an inner diameter of from 3.1 mm to 3.6 mm.

## Revendications

1. Kit prêt à l'emploi pour la fabrication d'une orthèse d'avancée mandibulaire (1) en deux parties, destinée à empêcher le ronflement et/ou l'apnée obstructive du sommeil, comprenant
(A) une partie inférieure (2) et une partie supérieure (3), constituées chacune d'une coque (2a, 3a) de modelage résistante à la flexion, respectivement ouverte vers la mâchoire inférieure ou la mâchoire supérieure lorsque l'orthèse est utilisée, laquelle contient une matière de remplissage thermoplastique (4, 5) postformable à la manière d'un appareil dentaire pour correspondre à la mâchoire supérieure et à la mâchoire inférieure humaines, les deux coques comportant sur leurs faces extérieures un ou plusieurs boutons de fixation pour la fixation d'un support de protrusion, lequel est fixé de manière à pouvoir pivoter sur un bouton de fixation de la coque pour mâchoire inférieure et de la coque pour mâchoire supérieure, respectivement, et ramène la mâchoire inférieure dans une position antérieure,
(B) deux jeux de deux ou plusieurs supports de protrusion (10) de longueurs différentes.
**caractérisée en ce que** le support de protrusion est stable à la dilatation ou à l'écrasement.

2. Kit prêt à l'emploi selon la revendication 1, **caractérisée en ce que** les boutons de fixation présentent un diamètre compris entre 2,0 et 5,0 mm, préférentiellement entre 3,0 et 4,5 mm, et en particulier égal à 4 mm environ.

3. Kit prêt à l'emploi selon la revendication 1 ou la revendication 2, **caractérisée en ce que** l'espacement entre la face intérieure du fond de la coque pour mâchoire supérieure (23) et la face intérieure du fond de la coque pour mâchoire inférieure (22) est en état d'assemblage inférieure à 8 mm, préférentiellement compris entre 1 et 5 mm, et en particulier égal à 2 mm environ.

4. Kit prêt à l'emploi selon l'une des revendications 1 à 3, **caractérisée en ce que** l'écartement des dents est compris entre 2 et 4 mm, et est préférentiellement égal à 3 mm environ lorsque l'orthèse est appliquée à l'avant des dents.

5. Kit prêt à l'emploi selon l'une des revendications 1 à 4, **caractérisée en ce que** la matière de remplissage (4, 5) est un polymère ou un copolymère biocompatible, toxicologiquement inoffensif, plastiquement déformable à la chaleur, préférentiellement en dessous de 70°C, en particulier entre 40 et 65°C, et qui se modèle étroitement sur une forme définie avant de conserver ladite forme après refroidissement.

6. Kit prêt à l'emploi selon l'une des revendications 1 à 5, **caractérisée en ce que** les supports de protrusion sont en polyoxyméthylène.

7. Kit prêt à l'emploi selon l'une des revendications 1 à 6, **caractérisée en ce que** les supports de protrusion dont la longueur est inférieure à l'espacement entre deux boutons de fixation, sont respectivement reliés au bouton de fixation avant de la partie supérieure et au bouton de fixation arrière de la partie inférieure, la mâchoire inférieure étant amenée en position antérieure par traction.

8. Kit prêt à l'emploi selon la revendication 7, **caractérisée en ce que** les coques (2a, 3a) résistantes à la flexion comportent respectivement sur chacune de leurs deux faces extérieures deux boutons de fixation d'espacement compris entre 20,0 et 30,0 mm, préférentiellement entre 22,0 et 28,0 mm, et en particulier égal à 26,7 mm environ.

9. Kit prêt à l'emploi selon l'une des revendications 1 à 8, **caractérisée en ce que** les supports de protrusion présentent chacun une longueur totale comprise entre 15,0 et 35,0 mm, préférentiellement entre i7,0 et 31,0 mm.

10. Kit prêt à l'emploi selon l'une des revendications 1 à 6, **caractérisée en ce que** les supports de protrusion dont la longueur est supérieure à l'espacement entre deux boutons de fixation, sont respectivement reliés au bouton de fixation arrière de la partie supérieure et au bouton de fixation avant de la partie inférieure, la mâchoire inférieure étant amenée en position antérieure par traction.

11. Kit prêt à l'emploi selon la revendication 10, **caractérisée en ce que** les coques (2a, 3a) résistantes à la flexion comportent respectivement sur chacune de leurs deux faces extérieures deux boutons de fixation d'espacement compris entre 10,0 à 20,0 mm, préférentiellement entre 12,5 ét 18,0 mm, et en particulier égal à 15,6 mm environ.

12. Kit prêt à l'emploi selon l'une des revendications 1 à 6, **caractérisée en ce que** les supports de protrusion présentent chacun une longueur totale comprise entre 15,0 et 30,0 mm, préférentiellement entre 17,0 et 27,0 mm.

13. Kit prêt à l'emploi selon l'une des revendications 1 à 12, **caractérisée en ce que** le support de protrusion présente à chacune de ses deux extrémités un évidement annulaire pour la réception d'un bouton de fixation sur la coque (2a, 3a) résistante à la flexion respective.

14. Kit prêt à l'emploi selon la revendication 13, **caractérisée en ce que** l'évidement annulaire est étagé et présente un diamètre intérieur compris entre 3,8 et 4,2 mm sur un côté et un diamètre intérieur compris entre 3,1 et 3,6 mm sur l'autre côté.
